Europäisches Patentamt

European Patent Office

Office européen des brevets

(11) Publication number : **0 484 057 A2**

## (12) EUROPEAN PATENT APPLICATION

(21) Application number : **91309856.2**

(22) Date of filing : **24.10.91**

(51) Int. Cl.$^5$ : **A61L 33/00**

(30) Priority : **02.11.90 US 608453**

(43) Date of publication of application :
**06.05.92 Bulletin 92/19**

(84) Designated Contracting States :
**BE DE FR GB IT NL**

(71) Applicant : **THE DOW CHEMICAL COMPANY**
**2030 Dow Center Abbott Road**
**Midland, MI 48640 (US)**

(72) Inventor : **Walles, Wilhelm E.**
**6648 River Road**
**Freeland, Michigan 48623 (US)**

(74) Representative : **Burford, Anthony Frederick et al**
**W.H. Beck, Greener & Co. 7 Stone Buildings Lincoln's Inn**
**London WC2A 3SZ (GB)**

(54) Antithrombogenic surfaces, their preparation, and materials therefore.

(57) An aqueous paint used to coat a solid substrate to render the surface thereof antithrombogenic comprises an antithrombogenic agent, a non-blood leachable organic polymeric binder and, optionally ; a spacer material. Preferably, the aqueous paint contains water, a base, heparin, ethylene acrylic acid copolymer, and colloidal silica. A polymeric resin substrate can be sulfonated prior to coating and/or a coated substrate can be treated with a non-blood leachable bonding agent, especially glutaraldehyde.

EP 0 484 057 A2

The present invention relates to the preparation of antithrombogenic surfaces and more particularly to an aqueous paint which may be coated onto the surface of medical devices in order to render those devices antithrombogenic and compatible with blood.

Extensive investigations have been undertaken over many years to find materials that will be biologically and chemically stable towards body fluids.This area of research has become increasingly important with the development of various objects and articles which can be in contact with blood, such as artificial organs, vascular grafts, probes, cannulas, and catheters.

Artificial materials are being increasingly used as blood contact devices and may be subject to potential generation of thrombi. When blood contacts a foreign material, a complex series of events occur. These involve protein deposition, cellular adhesion and aggregation, and activation of blood coagulation schemes. Considerable research has been focused on this blood-material interaction in the last twenty years. The overall objective of this research has been to minimize the potential for thrombi formation on the foreign materials, such as the device when introduced into the body upon contact with blood.

Accordingly, such medical devices are commonly referred to as antithrombogenic devices. Likewise, the term antithrombogenic agent or material refers to any material which inhibits thrombi formation on its surface, such as reducing platelet aggregation, dissolving fibrin, enhancing passivating protein deposition, or inhibiting one or more steps within the coagulation cascade.

Early work on antithrombogenic agents was done by Gott. The method used by Dr. Gott comprised treating a graphited surface first with Zephiran (benzalkonium chloride) and then with heparin. Materials treated in this way were nonthrombogenic in vivo for long periods of time. The major disadvantage, however, with these materials, was that the method could only be practiced on rigid plastics and a need still exists for a suitable flexible antithrombogenic plastic, as well as a method of producing the same. A recent book on the subject is Heparin, D. A. Lane and U. Undahl, CRC Press Inc., 1989.

Various materials have been devised for producing such a material. Most involve the use of quaternary amines in combination with heparin. First, quaternary amines were bound to the surface of a polymer and heparin was subsequently ionically bonded thereto. See, for example, Leining et al US-A-3,617,344. Later, Grotta in US-A-3,846,353 suggested complexing heparin with a quaternary amine on the polymer surface. In US-A-4,367,749; US-A-4,349,467; US-A-4,302,368 and US-A-4,116,989 a series of quaternary ammonium salts are disclosed which may be used to affix the corresponding quaternary amine to the surface of a polymer for subsequent ionic bonding to a negative ion of sodium heparin which may, for example, have attached sulfate and sulfonate groups.

A competing system involves the use of polyalkyleneimines in combination with an anti-thrombogenic agent such as heparin. Early work on this system was done by Love et al. and is represented by US-A-3,616,935, which discloses use of polyalkylene-imines to irreversibly adsorb the antithrombogenic agent to the intended surface through the formation of ionic bonds. US-A-4,639,339 discloses use of a sulfonated polyethyleneimine either alone or in combination with a heparin salt.

All of the above-mentioned systems are leachable ones wherein the heparin is ionically bonded to the polymer surface. Controversy has existed for years as to whether heparin should be (or should not be) gradually leached from the polymers at the blood interface in order to exert its anticoagulant effect. As stated in Ebey et al., "Heparinization of Medical Grade Polyurethanes", Journal of Biomaterials Applications, Vol. 2, pp. 475-519 (1988), "If heparin must be leached, the useful life of the material is limited. If bonded heparin can exert its anticoagulant effect without being eluted into the blood, then, the goal of long-term thromboresistance appears possible to reach." Ebey et al. concludes that, in fact, thromboresistance can exist in the absence of leaching of heparin and the authors go on to discuss various methods for chemically binding heparin to medical grade polyurethanes. Antithrombogenic polyurethanes are also discussed in the patent literature. See, for example, Solomon et al. US-A-4,521,564 and Yen et al. US-A-3,755,218 and US-A-3,853,804.

The work done on preparation of heparinized polymers as thromboresistant biomaterial has been summarized by Wilson in "Hemocompatible Polymers: Preparation and Properties", Polym.-Plast. Technol. Eng., Vol. 16, pp. 119-208 (1981). From the literature it is known furthermore, that some synthetic materials by themselves are antithrombogenic, particularly those with negatively charged molecular groups, such as ethyleneacrylic acid copolymers (EAA) and their esters and salts, synthetic materials which contain crotonic acid copolymers, and sulfonated synthetic materials which contain $SO_3H$ groups. See, for example, Sawyer US-A-3,886,947 which discloses a non-thrombogenic catheter made of a copolymer of an acidic material and ethylene; Boisson et al. US-A-4,568,725 which discloses anticoagulating polymers or copolymers containing $SO_3H$ and $SO_3R$ wherein R is a radical derived from argine, and the background discussion in Braun US-A-4,265,928.

The Braun patent discloses an antithrombogenic retentive catheter having a coating applied by immersing the catheter in pyridine or a toluol/tetra-hydrofuran solution of a copolymerizate of ethylene with acrylic acid (or esters or salts thereof) and drying. As such Braun relates to a coating or paint, albeit an organic based one, which is used to treat a polymeric medical device to render it antithrombogenic agent, and relies solely on the antithrombogenic nature of the EAA copolymer itself.

Feijen et al. US-A-4,634,762 discloses an aqueous, blood compatible, paint in the form of a conjugate of an anticoagulant and protein in an aqueous medium. Preferred are heparin-albumin complexes wherein a coupling agent such as 1-ethyl-3-dimethyl-aminopropyl-carbodiimide is used to form amine linkages between the heparin and albumin. The result is a conjugate which may be attached to a substrate surface either in association with a cross-linking operation or by being absorbed thereto.

While the system of Feijen et al. is an improvement on those of the past, it still has a number of drawbacks. Thus, as pointed out in Feijen et al. US-A-4,634,762, it is necessary to separate the formed conjugate from free protein and heparin that remain unreacted. The process for doing so is complex and time consuming. It would, therefore, be desirable to produce an aqueous, blood compatible, paint in a more efficient manner.

Accordingly, the need exists for improved antithrombogenic surface treatment materials and for a more efficient methods of preparing the materials.

That need is met by the aqueous paint, which upon drying is blood compatible, of the present invention. The paint of the present invention has as its primary ingredients: water, an antithrombogenic agent, an organic polymeric binder which is compatible with the antithrombogenic agent and is not leachable in acidic solutions, and optionally a material which is compatible with the antithrombogenic agent and organic binder and which functions to provide space in a layer of the paint so that the antithrombogenic agent can contact the blood.

The aqueous paint may be applied to any solid substrate which comes into contact with blood to render that surface antithrombogenic. Most medical devices are made of metal, glass, plastic, or combinations thereof. Thus metal medical devices and glass medical devices (or parts thereof) may be turned into antithrombogenic medical devices by coating them with the aqueous paint of the present invention and drying. However, the aqueous paint is most applicable for use on medical devices (or parts thereof) made of polymeric resin materials, including flexible plastic medical devices.

Antithromnogenic agent or material as used herein means any material which inhibits thrombus formation on its surface such as by reducing platelet aggregation, dissolving fibrin, enhancing passivating protein deposition, or inhibiting one or more steps in the coagulation cascade. An antithrombogenic agent may be heparin or sulfated chitosan or any one of a number of other materials such as extracts from lungs, liver and intestines, prostaglandins, urokinase, streptokinase sulfated polysaccharide, albumin and mixtures thereof. Preferred is heparin. Heparin is a term known in the art as referring to a family of straight chain anionic polysaccharides which contain unfractionated heparin (that is a heterogeneous mixture of more than 30 molecules of varying molecular weight extending from 1,200 to 40,000 daltons) as well as heparin fractions such as commercially available low molecular weight heparin fractions.

The non-leachable binder is any organic resin which is compatible with the antithrombogenic agent which when coated onto a surface and dried adheres to the surface, and is not leachable in acidic solutions but preferably is soluble in basic solutions. Preferably an acrylic acid copolymer such as an ethylene acrylic acid (EAA) copolymer having from 75 to 95 percent by weight ethylene content and 5 to 25 percent acrylic acid content is used. Other acrylic acid copolymers may also be used as long as the copolymer is pH sensitive from a solubility standpoint. A particular advantage of EAA is that it becomes water insoluble, and thus non-leachable in blood at the slightly acidic pH of blood. On the other hand EAA is highly soluble in alkaline solutions. This permits processing of the aqueous, blood compatible, paint under alkaline conditions, but once the paint has been coated onto a solid substrate, dried, and brought into contact with blood, its components are non-leachable. Still, the EAA may be redissolved in an alkaline solution, making it possible to wash the equipment and clean the solid substrate, even after deposition.

The spacer material is also preferably non-leachable under slightly acidic conditions. Preferred is a colloidal silica. While colloidal silica is somewhat brittle and delicate, it has the advantage of being highly compatible with EAA, it is soluble in water at approximately the same pH range as EAA, it is insoluble at the slightly acidic pH of blood, and it serves nicely as a spacer material which creates on the substrate an even array of solid particles amongst which the antithrombogenic agent is dispersed and bonded by the EAA. Other colloidal materials may also be used as long as they are salt free and have similar non-leachable properties. They include, for example, colloidal aqueous suspensions of aluminum and titanium oxides and hydroxides.

Finally, the water of the aqueous paint may be just that, that is pH 7 distilled water; although, preferably it has a base added to it so that the aqueous mixture is alkaline in pH. Ammonia compounds are preferred as the base since $NH_3$ escapes from the paint upon drying thus giving a better paint than if sodium or other inorganic bases are used. As mentioned because of the solubility of EAA and colloidal silica in that range, the paint is easily handled and does not fully set-up until coated onto a substrate, dried, and contacted with blood. Thus, the aqueous paint of the present invention preferably comprises water, base, heparin, ethylene acrylic acid copolymer, and optionally colloidal silica.

Among the polymeric resin materials which may be used as the solid substrate are polyolefins, polyamides, polyurethanes, polyesters, polycarbonates, polysiloxanes, polyacrylics, polystyrene, polytetra-fluoroethylene, polyvinylchloride, and natural and synthetic rubber. In most instances it is desirable to sulfonate the surface of the polymeric resin material prior to coating it with the aqueous paint.

Sulfonation may be accomplished for example by using the process and apparatus disclosed in US-A-4,902,493 and US-A-4,915,912.

Medical devices or parts thereof, whether it be metal, glass or sulfonated plastic, may be rendered antithrombogenic by coating it with the aqueous, blood compatible, paint of the present invention. Other solid substrates, and not just medical devices or parts thereof, may be rendered antithrombogenic by the method of the present invention which involves coating a surface of that substrate with the aqueous paint and drying.

Alternatively, the method of the present invention also involves the step of further treating the coated substrate with a bonding agent, such as a dialdehyde, such as, glutaraldehyde, which upon drying or curing helps adhere the paint to the solid substrate. As with the components of the aqueous paint, the bonding agent should be non-leachable in blood and otherwise safe for use with medical devices. The result is a coated medical device having improved antithrombogenic properties.

The antithrombogenic medical devices of the present invention fall into the permanently thrombo-resistant category in that the antithrombogenic agent is trapped by the non-leachable binder, and thus can exert its anticoagulant effect without being eluted into the blood. Long term thromboresistance is achieved.

Another advantage of the present invention is that the spacer material in the aqueous paint and on the resultant antithrombogenic surface, evenly disperses the antithrombogenic agent. At the same time, because of its particle size, the spacer material insulates and protects the binder-bound agent (which is found in the interstices of the spacer material) until contact with the contacting fluid, such as blood.

The paint can be made up with a wide range of concentrations of the individual constituents. For example, on a percent by weight basis the paint can comprise: water 50 to 99 percent, antithrombogenic agent 25 to 0.3 percent, organic binder 25 to 0.3 percent, and optionally spacer material to 25 percent. Preferably for ease and quickness of application the following ranges are used, water 10 to 98 percent, antithrombogenic agent 20 to 1 percent, organic binding agent 20 to 1 percent and spacer to 10 percent.

Accordingly, it is an object of the present invention to provide an aqueous, blood compatible, paint; antithrombogenic surfaces made by coating the surface with the aqueous paint; antithrombogenic medical devices coated with the aqueous paint a method for preparing antithrombogenic surfaces and antithrombogenic medical devices and a method for preparing the paint.

In the most preferred embodiment of the present invention, the aqueous paint of the present invention contains water, base, heparin, ethylene acrylic acid copolymer, and colloidal silica. Antithrombogenic agents other than or in addition to heparin may be used. Non-leachable binders, other than or in addition to acrylic acid copolymers may be used as long as they are non-leachable at blood pH. Other non-leachable spacer materials may be used together or in place of colloidal silica. Thus, the aqueous paint is essentially a mixture in alkaline solution of the antithrombogenic agent, a non-leachable binder for it, and non-leachable spacer material. After being coated onto the intended surface, the antithrombogenic agent is dispersed and bound by the binder amongst the arrayed particles of spacer material. The result is a surface having the essentially permanent antithrombogenic properties discussed above.

The aqueous paint of the present invention is useful in preparing antithrombogenic medical devices. Because the antithrombogenic properties are essentially permanent, it is particularly useful for in-dwelling or permanently attached medical devices such as artificial organs, vascular grafts, probes, cannulas, catheters, blood filters, blood sensors, biosensors, blood oxygenators, dialyzers, and tubing. While such medical devices often contain metal or glass parts to which the aqueous paint of the present invention readily adheres, many are fabricated of or contain polymeric resin materials.

Polymeric resin materials such as polycarbonates, polyurethanes, polysiloxanes and polyolefins are not as easily coated with the aqueous paint of the present invention. Accordingly, it is desirable to sulfonate the surfaces of the polymeric resin materials using for example the process and apparatus of US-A-4,902,493 and US-A-4,915,912. There is disclosed in those patents a system for the production of sulfur trioxide reagent and the use of the sulfur trioxide reagent so generated for the surface treatment of polymeric resin materials. The system there disclosed is particularly useful with medical devices, where the surfaces which contact the blood or other body fluid of a patient must be water-wettable.

Wettability is needed to prevent air bubbles from sticking to a surface and ending up in a patient's blood, or causing irregular flow through a tube or the like. Wettability is also important for preventing blood from sticking to or coagulating on a surface. However, most, if not all, of the polymeric resin materials utilized in such medical devices have hydrophobic surface properties. Sulfonation of such surfaces make the surfaces hydrophilic and thus water wettable.

When the surfaces of the polymeric resin materials have been sulfonated, it is also possible to render them antithrombogenic by coating them with the aqueous paint of the present invention, and drying. It should be recognized that there are some polymeric materials or other treatment for polymeric materials other than sulfonation which may be useful. Alternatively, a further treatment with a bonding agent such as glutaraldehyde is used to insure an even better, more permanent adherence of the paint to the surface of the solid substrate. To prepare a preferred paint of the invention, the binder, such as an ethylene acrylic acid copolymer, is dissolved in water containing $NH_3$ to render it basic. This mixture is then mixed with the antithrombogenic agent such as heparin in water and then optionally with the spacer material such as colloidal silica in water. The paint will set up when dried. Drying can be accelerated by heating to a temperature of 40 to 60°. The $NH_3$ will evaporate changing the pH of the binder.

The following examples are illustrative of the aqueous, blood compatible, paint of the preferred embodiment and the antithrombogenic nature of the surfaces so produced:

Example 1 and Control Tests

A thromboresistance test was conducted to evaluate the interaction of the antithrombogenic paint surface with circulating canine blood. An _ex vivo_ screening method was employed. A healthy, random-source dog obtained from a USDA licensed supplier and acclimated to the laboratory was selected for this study. The dog was identified by its USDA tag number and individually housed in a kennel run. Commercial dog food was provided daily and tap water freely given. Blood was drawn for analyses as deemed necessary. Test/control samples were approximately 23 mm in diameter in order to fit the _ex vivo_ test chambers required. Two samples were secured inside each flow-through plastic chamber device as part of an arteriovenous shunt.

The dog was placed under general anesthesia in a routine fashion and the femoral vein and artery were isolated via surgical cut-down. Intravenous tubing was connected to the artery and vein to create an external arteriovenous shunt in conjunction with the chambers. Exposure of the sample surfaces to circulating blood in the manner for various time intervals was immediately followed by rinsing with saline. The samples had, before the test, been coated with an aqueous solution containing the constituents shown in Table I as parts by weight and dried to form an adherent coating. In sample numbers 5-14 the aqueous solution contained 20 percent by weight of the constituents and in sample number 2, 21 percent by weight of the constituents. All test/control samples were subsequently refrigerated and prepared for scanning electron microscopy (SEM) examination and photomicrography to determine the extent of platelet deposition on blood-exposed surfaces. The higher the number of platelets, the lower the thromboresistance performance. Total blood exposure time was 5 minutes. The samples used in this example are described in Table I as follows:

## TABLE I

### Thromboresistance Test Samples

The substrate surfaces were made from polystyrene film and sulfonated with $SO_3$ in air. The constituents are reported in parts by weight.

| Sample Number | Treatment Description |
|---|---|
| 1 | Sulfonated only (control) |
| 2 | Sulfonated plus 16 parts Heparin, 44 parts EAA*, 39 parts silica |
| 5 | Sulfonated plus 40 parts Heparin, 10 parts EAA |
| 6 | Sulfonated plus 60 parts Heparin, 40 parts EAA, 20 parts silica |
| 7 | Sulfonated plus 40 parts Heparin, 40 parts EAA, 20 parts silica |
| 8 | Sulfonated plus 40 parts Heparin, 30 parts EAA, 30 parts silica |
| 9 | Sulfonated plus 40 parts Heparin, 20 parts EAA, 40 parts silica |
| 10 | Sulfonated plus 50 parts Heparin, 40 parts EAA, 10 parts silica |
| 11 | Sulfonated plus 5 parts Glucosamine, 5 parts Heparin |
| 12 | Same as 11 but with higher level of sulfonation |
| 13 | Sulfonated plus 5 parts Hexamethyldiamine, 5 parts Heparin |
| 14 | Same as 13 but with higher level of sulfonation |

* Ethylene acrylic acid copolymer

The results of the study are summarized in Table II as set forth below. In general, all but samples 7, 8 and 9 outperformed the sulfonated control.

## TABLE II

| Sample Number | Platelet Adhesion |
|:---:|:---|
| 2 | Much better than the control (sample #1) |
| 5 | The best performing treatment with no visible platelets after 5 minutes |
| 6 | Much better than the control |
| 7 | Same as the control |
| 8 | Same as the control |
| 9 | Much worse than the control |
| 10 | Slightly better than the control |
| 11 | Much better than the control |
| 12 | Much better than the control |
| 13 | Much better than the control |
| 14 | Much better than the control |

Example 2 and Control Test

An in-vitro thrombogenicity study was conducted via the plate method as described below. The samples employed in this study are as set forth in Table I as shown above.

A 4 cm² section was cut from each of the twelve sheets of samples as described in Table I and transferred to separate sterile Petri dishes. Approximately 60 ml of whole rabbit blood was obtained by cardiac puncture of an anesthetized rabbit. The blood was immediately placed onto the surface of two 15 x 150 mm Tryptic Soy Agar (TSA) plates (30 ml of blood per plate). Half of the samples were placed onto each plate with the coated side of the samples in contact with the blood. A section of acid washed glass tubing was also added to each plate as a positive control. The plates were incubated at 37°C for 20 minutes.

The test was repeated by embedding one edge of each sample section into the agar surface. This permitted exposure of both the coated and uncoated surfaces to the blood. The blood collection and incubation were performed as described above. Following incubation, the sections were removed from the plates using forceps. Upon removal, each of the sample sections were observed for evidence of clot adherence.

The results indicated that none of the samples, including the sulfonated control (sample #1) had any signs of clot adherence after 20 minutes. All of the samples as set forth in Table I unexpectedly did not show clot adherence.

Example 3 and Control Test

The purpose of this study was to screen or compare the thromboresistance of various types of tubing materials. It is most useful for comparing the relative thromboresistance of groups of materials or tubings with and without nonthrombogenic or anticoagulant coating. These samples were prepared from tubing approximately 120 cm in length with an internal diameter of approximately 25 mm. Segmented samples of glass tubing were used as a positive control. The samples are set forth in Table III below. The samples coated with heparin only were prepared by washing the tubing with a 10 percent by weight solution of heparin in water (Samples 3 and 7). The paint of the present invention (Samples 4 and 5) was applied as an 18 percent by weight aqueous solution.

7

## TABLE III

The substrate surfaces were thermoplastic polyurethane (TPU) and polyvinylchloride (PVC) and sulfonated by exposure to a solution of 0.5 percent SO$_3$ in Freon 113 for 15 seconds.

| Sample Number | Treatment Description |
|---|---|
| 1 | TPU; untreated |
| 2 | TPU; sulfonated only |
| 3 | TPU; sulfonated plus Heparin |
| 4 | TPU; sulfonated plus 16 parts Heparin, 44 parts EAA, 40 parts silica |
| 5 | PVC; untreated |
| 6 | PVC; sulfonated only |
| 7 | PVC; sulfonated plus Heparin |
| 8 | PVC; sulfonated plus 16 parts Heparin, 44 parts EAA, 40 parts silica |

A healthy random source dog weighing approximately 10 to 20 kg was fasted overnight. Following anesthesia, the femoral artery on one side was surgically exposed and a test tubing sample, pre-filled with sterile 0.9 percent saline, introduced into the artery. The insertion was facilitated by fitting an adapter to one end and a syringe to the other. After insertion, the syringe was removed and the blood allowed to freely flow for five seconds. The tubing was then clamped at both ends and removed from the artery. The samples were immersed in a 37°C water bath. Six segments of the tube (15 cm each) were segmented by clamping and cutting the tube and the tubing was removed, one section at a time, at time intervals. The clamps were removed and the blood emptied onto a section of white absorbent paper and examined for the presence of clots.

The endpoint of the test was the time required for the formation of clots 2 cm or more in length. Samples were compared by the length of time required to produce the 2 cm clots. The samples were studied for total clot formation relative to a known positive glass control as well as other control samples such as the untreated and sulfonated only samples. A positive result was determined by a clot of greater than 2 cm. The total test time was 300 minutes.

The results of this study are set forth in Table IV as shown below:

TABLE IV

| Sample Number | Time to Clotting |
|---|---|
| Glass Tubing (positive control) | after 10 min., clot >2 cm |
| 1 (TPU) | after 15 min., clot >2 cm |
| 2 (TPU) | after 30 min., clot >2 cm |
| 3 (TPU) | no clot after 300 min. |
| 4 (TPU) | no clot after 300 min. |
| 5 (PVC) | after 40 min., clot >2 cm |
| 6 (PVC) | after 30 min., clot >2 cm |
| 7 (PVC) | no clot after 300 min. |
| 8 (PVC) | no clot after 300 min. |

Most nontreated/nonheparinized plastics fall within an intermediate group with clotting times ranging from 23 to 173 minutes. In general, these results showed increased thromboresistance performance proceeding from untreated, to sulfonated, to heparinized samples for both TPU and PVC.

Example 4

A biocompatibility study for cytotoxicity using on agarose overlay was completed on the samples described in Table I above according to the following procedure. A monolayer of L-929 Mouse Fibroblast cells were grown to confluency and overlaid minimum essential medium supplemented with serum, antibiotics, neutral red, and agarose. The test article was placed on the solidified overlay surface.

Following incubation for 24 hourse, the culture was microsconically examined for evidence of cell decolorization to determine the zone of cell lysis. Any decolorized zone present was examined microscopically to conform cell lysis. A "nontoxic" observation indicated that no change in cell morphology occurred in proximity to the test sample. Conversely, a "toxic" observation indicated death and/or degeneration of cells directly beneath the area of the test sample and possibly also within a zone extended beyond the test sample. Where a zone of lysis was observed, the distance from the edge of the sample to the edge of the zone was measured and reported in millimeters (mm).

The results are set forth in Table V as shown below:

## TABLE V

| Sample Number | Observation | Zone of Lysis (mm) |
|---|---|---|
| 1-11 | Nontoxic | 0 |
| 12 | Toxic | – – – |
| 13 | Nontoxic | 0 |
| 14 | Toxic | – – – |
| Negative Control | Nontoxic | 10 |

The results indicated that all the test samples in Table I were noncytotoxic except for sample number 12 and sample number 14. These two samples were highly sulfonated with glucosamine and diamine formulations which is consistent with bio-toxic data previously generated.

## Claims

1. An aqueous paint suitable for the preparation of antithrombogenic surfaces, comprising water, an antithrombogenic agent, and an organic polymeric binder which is substantially nonleachable in blood.

2. A paint of Claim 1, wherein said antithrombogenic agent is heparin or sulfated chitosan.

3. A paint of Claim 1 or Claim 2, wherein said binder is an acrylic acid copolymer.

4. A paint of Claim 3, wherein said acrylic acid copolymer is an ethylene acrylic acid copolymer having an acrylic acid content of between 5 and 25 percent.

5. A paint of any one of the preceding claims, including in addition a spacer material which is substantially nonleachable in blood.

6. A paint of Claim 5, wherein said spacer material is colloidal silica.

7. A paint of any one of the preceding claims, which has a basic pH.

8. A paint of Claim 7, comprising ammonia, heparin, an ethylene acrylic acid copolymer having an acrylic acid content of between 5 and 24 percent, and colloidal silica.

9. A paint of any one of the preceding claims, comprising 50 to 99 wt. % water; 25 to 0.3 wt. % antithrombogenic agent; 25 to 0.3 wt. % organic binder; and, optionally, up to 25 wt. % spacer material.

10. A paint of Claim 9, comprising 10 to 98 wt. % water; 20 to 1 wt. % antithrombogenic agent; 20 to 1 wt. % organic binder; and, optionally, up to 10 wt. % spacer material.

11. A method for rendering antithrombogenic a surface of a solid substrate comprising coating said surface with an aqueous mixture of water, an antithrombogenic agent, and an organic polymeric binder which is substantially nonleachable in blood, and drying said coating to provide a coated surface of said substrate.

12. A method of Claim 11, wherein said paint is as claimed in any one of Claims 2 to 10.

13. A method of Claim 11 or Claim 12, further including the additional step of treating said coated surface with a bonding agent.

14. A method of Claim 13, wherein said bonding agent is glutaraldehyde.

15. A method of any one of Claims 11 to 14, wherein said solid substrate is a polymeric resin material and said method further includes sulfonating said surface prior to coating with said aqueous mixture.

16. The use in the preparation of antithrombogenic surface coatings of a paint as claimed in Claim 1.

17. A use of Claim 16, wherein said paint is as claimed in any one of Claims 2 to 10.

18. An antithrombogenic medical device comprising a solid substrate having coated thereon a mixture of an antithrombogenic agent, and an organic polymeric binder which is substantially nonleachable in blood.

19. A medical device of Claim 18, wherein said solid substrate is selected from polyolefins, polyamides, polyurethanes, polyesters, polycarbonates, polysiloxanes, polyacrylics, polystyrene, polytetrafluoroethylene, polyvinyl chloride, synthetic and natural rubber.

20. A medical device of Claim 18 or Claim 19, including in such mixture a spacer material which is substantially nonleachable in blood.

21. A medical device of any one of Claims 18 to 20, wherein said antithrombogenic agent, said organic binder and/or, if present, said spacer material is as specified in any one of Claims 2 to 8.

22. A medical device of any one of Claims 18 to 21, wherein said mixture was coated onto said substrate as a paint as claimed in any one of Claims 1 to 10.